Europäisches Patentamt

European Patent Office

Office européen des brevets

(11) Publication number: **0 524 360 B1**

## EUROPEAN PATENT SPECIFICATION

(45) Date of publication of patent specification: **30.11.94**

(51) Int. Cl.⁵: **C07K 15/00, A61K 37/36, A23K 1/165**

(21) Application number: **91402035.9**

(22) Date of filing: **22.07.91**

(54) **A somatotropin-based improved process for the farming of salmonids, particularly in seawater.**

(43) Date of publication of application:
**27.01.93 Bulletin 93/04**

(45) Publication of the grant of the patent:
**30.11.94 Bulletin 94/48**

(84) Designated Contracting States:
**GB**

(56) References cited:
EP-A- 0 166 444      EP-A- 0 213 357
EP-A- 0 239 075      EP-A- 0 377 540
EP-A- 0 387 457      EP-A- 0 438 929

(73) Proprietor: **EUROGENTEC S.A.**
**Campus du Sart-Tilman**
**Allée du Six Août**
**B6 Bldg**
**B-4000 Liège (BE)**

(72) Inventor: **Smal, Jean**
**9 rue de la Sablonnière**
**B-4031 Angleur - Liège (BE)**
Inventor: **Renard, André**
**37 avenue des Cerfs**
**B-4031 Angleur - Liège (BE)**

(74) Representative: **Gutmann, Ernest et al**
**Ernest Gutmann - Yves Plasseraud S.A.**
**3, rue Chauveau-Lagarde**
**F-75008 Paris (FR)**

**Description**

The present invention relates to a somatotropin-based process for improving the farming conditions of salmonids, particularly in seawater.

Salmonids are characterized by a life cycle involving both freshwater and seawater phases. Spawning and early life occur in freshwater (juvenile in freshwater is called a parr). Premature transfer of parr to seawater is lethal. Most salmonids species reach naturally a stage known as the smolt stage when the fish is capable of surviving in the sea, before to migrate into the ocean where most of its growth occurs.

This parr-smolt transformation (smoltification), which occurs only once a year, between february and may in the northern hemisphere, is a critical step in the life cycle of the fish and thus in its farming (the aquaculture of trout and salmon is one of the major intensive aquaculture in the world). For example, the production of S1 (= smoltification in less than 2 years from the date they hatched) or if possible SO smolts (= in less than 1 year from hatching) is regarded as highly desirable in atlantic salmon farming. The results usually reported are of less than 10 % S0 smolts, 40 to 70 % S1 smolts and the remain being S2 smolts.

The smoltification process involves complex physiological, anatomical and behavioural changes. These changes that take place during several months are triggered by changes in water temperature and day lenght, and involve numerous hormonal changes (Hoar, Fish Physiology, 11B, 275:343, 1988; Boeuf. La Pisciculture Française, 87, 28:52, 1987 & 88, 5:21, 1987). Among other hormones, somatotropin is involved in the plasma ion balance control of salmonids (ibid.). A recent paper discloses the optimisation of a somatotropin bioassay based on the measure of its acute effect on plasma ion lowering in rainbow trout after transfer from fresh water to seawater, independantly of effects on body size (Collie et al., Fish Physiology and Biochemistry, 7, 315:321, 1989).

Recent data showed that fishes which were implanted with cholesterol powder pellets compacted with a recombinant ovine growth hormone, which provided for a slow release of that growth hormone in salmonid species, provided them with an improved capability of adaptation to seawater.

The fishes that survived in sea water and which were maintained therein then also resumed their growth; Boeuf et al, C.R.Acad. sci, Paris, A310, Série III, 75:80, 1990). The authors refer to earlier studies in relation to the stimulation of the seawater adaptability of salmonids. Boeuf et al hypothesize that the use of their pelleted implants might represent a possible substitute for the long multiple injection procedures which had been suggested earlier by others, which procedures were considered as likely to entail chronical stress conditions and loss of appetite in the fishes so treated, in addition to other practical drawbacks.

Regardless of whether the substitute method suggested by Boeuf et al may indeed allow transfer of salmonids to sea water, its applicability under regular salmonid farming procedure remains doubtful having regard to the sophisticated techniques which the method would require : anesthesiation of the fishes, incision thereof and introduction of the compacted pellets in their peritoneal cavities. The inventions aims at obviating these difficulties and to provide a farming method both simple and efficient, applicable to salmonids, even out of their natural periods of smoltification where appropriate, that will allow high survival and growth rates of the fishes after their transfer to seawater.

A further object of this invention is to provide a process which provides for an increased survival rate and rapid capability of the treated animals to resume or undergo effective growth rate in the sea water farming step.

Another object of the invention is to provide procedures which will help the fish farmer to better plan his production, increase his productivity and reduce his production costs. Still another object of the invention is to avoid all complications related to the natural smoltification process, e.g. sensitivity to diseases and poor growth thereafter.

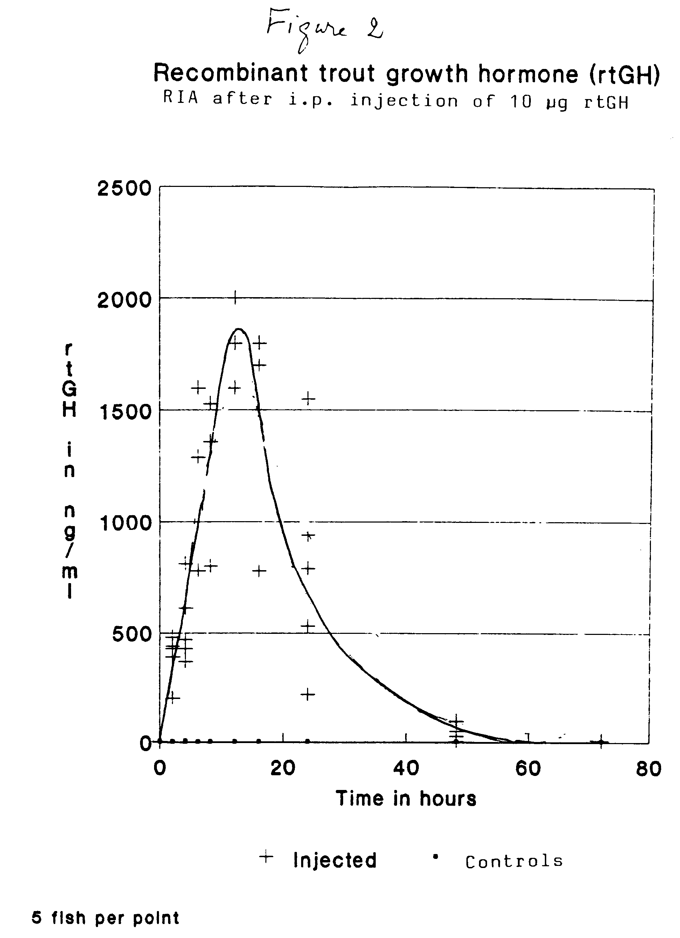

The process of the invention for the aquaculture of salmonids which comprises a farming step of these salmonids in a fresh water farm and another farming step in plain sea water for a time long enough to enable them to grow to the size finally sought, is characterized by a single administration of somatotropin in a form readily releasable in the blood circulation of said farmed salmonids prior to their transfer from the fresh water farm into said plain sea water, in an amount sufficient to induce in their blood circulation a supraphysiological pulse of somatotropin effective to cause resumption of the growth of said salmonids to said size finally sought during said farming step in plain sea water. The "supraphysiological pulse"as hereindefined refers to the transient intense peak - far more important than the endogenous naturally occuring pulses - that the in vivo concentration of somatotropin in the blood serum undergoes in the fishes, subsequent to the somatotropin administration, in accordance with the process of this invention. Fig. 2 hereafter provides an example of the supraphysiological pulse induced in trout administrered with a recombinant trout growth hormone. This pulse - generally a single one - is generally characterized by a rapid increase in time of the serum concentration of somatotropin and, after the passage through a peak, by

2

a decrease of that concentration which after a while tends to return to normal concentrations as measurable for endogenous somatotropins.

Reference will further be made hereinafter when appropriate and in a non limitative manner, to the drawings in which

. Fig .1 is indicative of concentrations in pg of somatotropin or trout growth hormone = tGH in serum of rainbow trouts (20-100g) reared under natural conditions of photoperiodism and temperature. Circulating tGH was assayed at different times of the year by radioimmunosassay (RIA) in 40 fishes.

. Fig.2 is illustrative of the variations characteristic of the supraphysiological pulse of the increased concentrations, in ng/ml, of a recombinant trout growth hormone (rtGH) in the serum of a rainbow trout injected intra-peritoneally with that rtGH, versus time (T) in hours after that injection (10 $\mu$g) : "+" signs are representative of mean values measured in groups of five treated trouts and the "dots" "." in groups of controls.

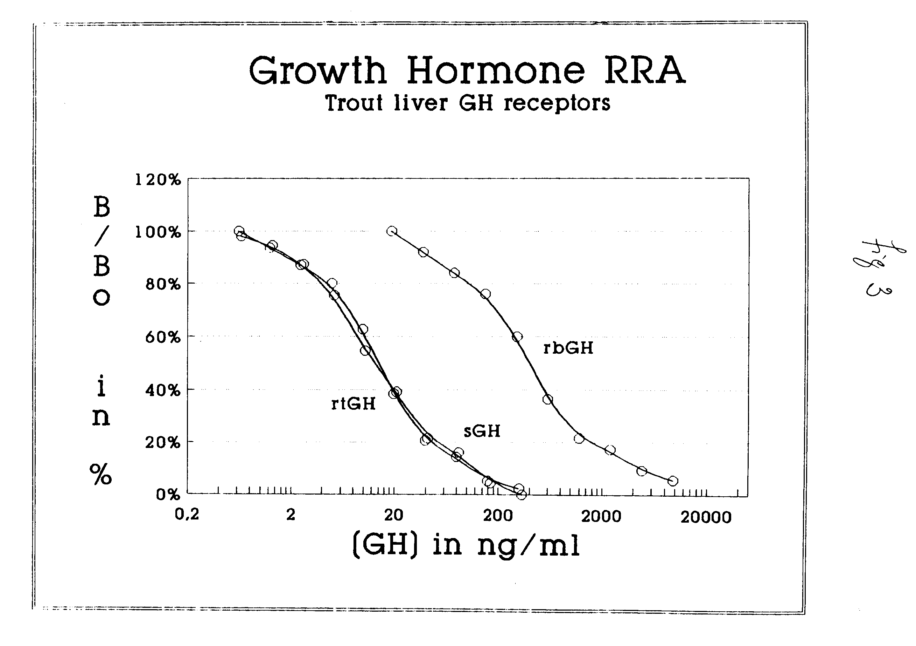

. Fig.3 comprises curves representative of competition curves obtained on trout liver membrane preparations, in radioreceptor-assays e.g. for the sake of evaluating the respective affinities of homologous, recombinant or heterologous somatotropins (or growth hormones) for receptors for one of them : trout growth hormone receptor.

The invention also relates to a composition of purified somatotropin (ST) for use in the above said process, mainly by parenteral injection (e.g. of an aqueous solution), oral (e.g. by addition of a somatotropin preparation into the food), dipping (immersion of the fish into a concentrated solution of a somatotropin preparation for at least 30 seconds) or bath treatment (direct addition of a somatotropin preparation to the tank containing the fish with or without water flow arrest to insure a contact with the somatotropin of at least 30 minutes) to allow efficient transfer of salmonids to seawater, independently of photoperiodism, water temperature and animal size.

Somatotropin refers to the family of proteins

. with a molecular weight of around 22 Kd produced by the pituitary gland of vertebrates, including fish, involved in the regulation of anabolic processes, and related to the Growth Hormone family defined in mammalian species (Nicoll, Endocr. Rev., 7, 169:203, 1986); for salmonid species, such hormone has been characterized in Chum salmon (Kawauchi et al, Arch. Biochem. Biophys., 244, 542:552, 1986), Coho salmon (Nicoll et al, Gen. Comp. Endocrinol., 68, 387:399, 1987) and rainbow trout (Rentier et al, DNA, 8, 119:127, 1989),

. and which can specifically bind to the receptors of the natural somatotropin (isolated from the pituitary gland of trout or salmon) present on the membrane of trout or salmon liver cells; the affinity of the somatotropin being measured by a radio-receptor assay (such assay has been described in Konakou Yao et al. (Gen. Comp. Endocrinol., 81, 72:82, 1991).

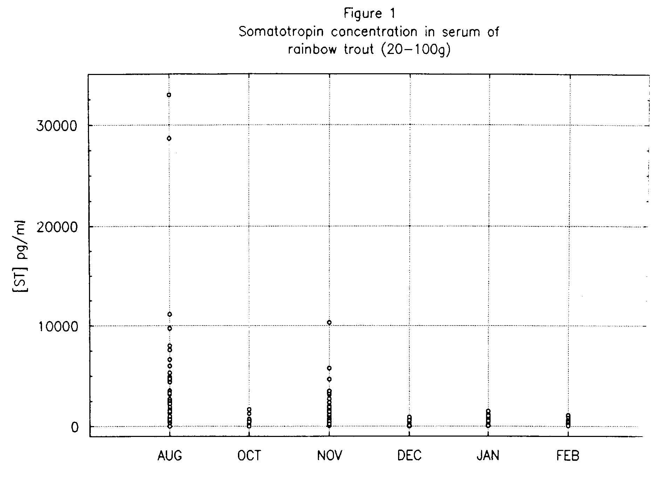

The endogenous somatotropin levels in salmonid blood are very variable (0-40 ng/ml, see figure 1), due to the pulsatility of its secretion by the pituitary as observed in all others vertebrates species (Jansson, Eden & Isaksson, Endocrin. Rev., 6, 128:145, 1985). By analogy with those species, the somatotropin natural pulse duration is very limited in time, usually under 3 hours, due to its fast clearance from the serum (ibid). Preferably the transfer of the treated fishes to sea water after the simple administration of somatotropin does not occur before the time at which said supraphysiological pulse (see for instance fig. 2) reaches its maximum, or even is over or completed and the blood concentrations in somatotropin (endogenous) are substantially back to normal.

The following figures are but illustrative of how the dosages of growth hormone (native GH or recombinant GH corresponding to that native GH) administered are to be adjusted to produce said supraphysiological pulse : e.g. the amount of somatotropin administered is adjusted such either that the supraphysiological pulse be over within the three days that follow the administration or that the peak of that pulse exceeds 50 ng, even 300 ng and whenever required 750 ng per ml of blood, or both.

It will be readily apparent that this "supraphysiological pulse" will vary depending on the fish species that are treated, on the nature of the somatotropin used (homogenous or heterologous) as well as on the selected routes of administration. The doses of somatotropin to be administered once should in each instance be determined experimentally, it being understood that the persons skilled in the art are considered to be able to determine, by upon exerting their normal skills, the different parameters, including the interval of time that will separate the effective administration of somatotropin to the fishes and their transfer to sea water, such as to achieve the goal sought by the invention, i.e. increase of survival rate and rapid capability of the treated animals to resume or undergo effective growth in sea water as a result of a single administration of that somatotropin.

By way of example the simple administration will often take place from 1 to 30 days, preferably from 3 to 20, e.g. from 10 to 14 days prior to sea transfer.

EP 0 524 360 B1

It will be understood that for the purpose of the further discussion the expression "homologous" as used with respect to the somatotropin pertains either to the native somatotropin of the fish species to be treated or to corresponding recombinant proteins, e.g. those including the expression product of a recombinant DNA including a DNA formed starting from corresponding native RNAS or RNA fragments. Consequently "heterologous" somatotropins correspond to native somatotropins from different fish species or even native mammalian or avian somatotropins or to corresponding "recombinant" somatotropins according to the definition provided hereabove, in relation to "homologous recombinant" somatotropins.

The process of the invention is applicable to any form of salmonids. Though applicable with special advantage to species of the salmo family, even more specifically to those salmonids which naturally undergo smolting (e.g. Salmo salar), it can also be used efficiently with other salmonids, e.g. those of the Oncorhynchus family.

Preferably the somatotropin for use in the composition of this invention is a salmonid somatotropin.

Preferably, the somatotropin used is identical or homologous to that of the fish administered with it: e.g. natural trout somatotropin or recombinant somatotropin having, as far as the somatotropin part thereof is concerned, substantially the same aminoacid sequence as the natural trout somatotropin, when said somatotropin is intended for administration to trout. But this is not an essential condition. For instance trout may be administered with purified somatotropin originating from salmon or tilapia, or even with somatotropin of mammalian origin, e.g. bovine somatotropin, or avian origin. Recombinant somatotropin can be substituted for the natural ones. The same observations extend to salmonid species other than trout , e.g. atlantic salmon.

The somatotropin concentrations used mentionned hereafter are relevant with respect to treatments of a salmonid species with homologous somatotropin (either natural or recombinant). When using heterologous somatotropins, their concentrations have to be corrected by their relative affinity for the receptors present in the liver cells of the recipient species. For example, bovine somatotropin has an affinity for the salmon hepatic somatotropin receptor which is 30 times lower than the affinity of the natural salmon somatotropin. In this invention, this somatotropin may be used but at a concentration 30 times higher than the salmon somatotropin to reach the same effect, as this can be appreciated from the curve relative shifts which appear in fig.2.

More generally the amounts of heterologous protein to be administered in accordance with the invention can be evaluated upon multiplying the suitable dosages that can be used with the homologous protein by a coefficient corresponding to the ratio of the affinity of a homologous somatotropin to the affinity of said heterologous somatotropin for somatotropin receptors as respectively measured in a radio-receptor-assay using natural receptors prepared from salmonid liver membranes.

The radioreceptor assay (RRA) for use in that evaluation is advantageously that disclosed by Kouakou Yao, Ping-De Nin, Florence Le Gac and Pierre Yves Le Bail et al in the article titled "Presence of Specific Growth Hormone Binding Sites in Rainbow Trout (Oncorhynchus mykiss) Tissues = characterization of the Hepatic Receptor" in "General and Comparative Endocrinology" 81, 72-82 (1991) whose contents shall also be deemed to be part of the present description where relevant.

Be it simply recalled here for the purpose of an easy understanding of the invention that the RRA may be outlined as follows :

. A sample of an homogenate prepared from a receptor rich organ (liver) of rainbow trout is incubated with an excess of rainbow trout growth hormone labelled with radioactive iodine;

. Similarly another sample of that homogenate is incubated with both the labelled rainbow trout growth hormone and the unlabelled growth hormone whose relative affinity for the rainbow trout receptors is to be determined, whereby a competition between the added unlabelled hormone and the labelled rainbow trout growth hormone for these receptors is induced.

The competition curves are obtained upon measuring the variation of the ratio of the radioactivity values (B) which remain in the homogenate after it was contacted with the unlabelled growth hormone under study to the radioactive value (Bo) measured on the homogenate sample which was incubated with the labelled trout hormone alone.

The curves of Fig.2 are illustrative of the respective affinities expressed by the variations of the B/Bo ratios on the axis of ordinates versus the concentration in ng/ml, of non-radioactively labelled hormone (chinook salmon growth hormone : sGH ; recombinant trout growth hormone : rtGH and recombinant ovine grow hormone : rbGH) in the presence of the trout receptors under study.

The value of the coefficient for use as a multiplication factor in the determination of the dosages of heterologous somatotropin for the treatment of determined fish species can be appreciated from the shift of the RRA curve obtained with the heterologous hormone with respect to the RRA curve obtained with the homologous hormone, at the 50% levels of the B/Bo ratios.

4

The composition of the invention is a composition in a form suitable for administration to fish, preferably by parenteral injection (e.g. intraperitoneally), orally, by bath or by dipping. The said composition comprises a somatotropin active in salmonids and when given once to the fish is generating a short supraphysiological pulse of somatotropin in their blood.

A preferred composition is in a liquid form or sufficiently fluid or dispersed form to allow its injection in fish, its addition to the food or directly to the water of the tank containing the fish. This composition may be prepared with lyophilised powder and an adequate buffer.

The invention pertains more particularly to a composition administrable to fish upheld in freshwater wherein the dose of somatotropin is adjusted both relative to the size of the fish for which it is intended and relative to the amount which can be administered to them by the appropriate route, at a level sufficient to generate a short supraphysiological pulse in the recipient fish, for example obtained by the i.p. injection of at least 0,03 $\mu$g but preferentially 0,1 $\mu$g to 0,25 $\mu$g per g of fish, that will allow within one day but preferentially after 10 to 14 days, the transfer of the fish to seawater with a survival rate which is cost effective for the fish farmer. Such transfer is characterized by a survival rate, one month after the treatment, much greater than the survival rate of untreated animals (the exact figure depends on the season the temperature and the animal weight but is usually greater than 25 %) and a sustained growth rate after the transfer (usually less than 10 % of stunted animals). Similar transfer without treatment and outside of the natural smoltification period will lead to high mortality and large proportion of stunted animals.

Suitable compositions to be administered by parenteral injection contain an amount of somatotropin adjusted, relative to the fish species to which it is to be administered, in a range (relative to the homologous somatotropin ) from 30 ng to 30 $\mu$g per gram of fish, preferentially from 0,10 to 0,25 $\mu$g.

Suitable compositions to be administered orally (e.g. together with food) contain an amount of somatotropin adjusted, relative to the fish species to which it is to be administered, in a range (relative to the homologous somatotropin) from 10 $\mu$g to 50 mg per gram of fish food.

Suitable compositions to be administered by dipping contain an amount of somatotropin adjusted relative to the fish species to which it is to be administered in a range (relative to the homologous somatotropin) from 25 mg to 10 g per litre of water. Dipping of the fishes normally lasts for at least 30 seconds.

Suitable compositions to be administered by bath treatment contain an amount of somatotropin adjusted, relative to the fish species to which it is to be administered, in a range (relative to the homologous somatotropin) from 2,5 mg to 1 g per litre of water. Maintaining of the fishes in that medium normally lasts for at least 30 minutes.

Additional preferred conditions, particularly for use in combination with the features recited above, which ought to be fulfilled are indicated hereafter in connection with examples.

One way to produce a ST is to start with pituitary glands isolated from a vertebrae animal, e.g. salmon or trouts, to mechanically break the cells and, for example, extract the hormone in suitable buffer, to purify the hormone by chromatography as described for example for Chum salmon in Kawauchi et al., 1987 (ibid.). Pituitary gland powder obtained by drying or lyophilisation of pituitary gland extracts may be used as such. Other conventional enrichment and purification techniques may also be used as far as the final preparation has no residual toxicity for fish and is characterized by a high potency of osmoregulation in terms of Na-K-ATPase activity stimulation and/or fish survival after transfer of treated animal in seawater.

Another way to produced the ST is to start with a bacterial or a yeast recombinant clone containing a plasmid with the ST gene, obtained by standard recombinant DNA technology methods from the messenger RNA isolated from pituitary gland, behind an adequate promotor allowing the high level expression of the protein into the cytoplasm, or, if a signal peptide is used, into the periplasm or the fermentation medium as described, for example, in European Patent Filing 894000047.0 (1989). If the somatotropin is produced as an insoluble material, it can be purified, after breaking of the cells, by a centrifugation step followed by denaturation in a strong chaotropic agent, dialysis and a chromatographic step as precedently described in Langley et al, Eur. J. Biochem., 163, 313:321, 1987. The final product is characterized by a low level of residual toxicity for fish and a high potency of osmoregulation in terms of Na-K-ATPase activity stimulation and/or fish survival after transfer of treated animal in seawater. The denaturation step may be omitted if the initial preparation is directly bioactive which is generally what is observed when the protein is excreted into the periplasm of the microorganism or into the fermentation medium.

To help and to facilitate the cloning, expression, renaturation and purification of the protein, to change the affinity of the hormone for its receptor, to change its stability in the fish tissue, in the food or in the water, such recombinant hormone may contain additional aminoacids at the carboxy- and amino-terminus or internally, may be deleted of some of its aminoacid or may be mutated, as far as the modifications are not preventing the binding of the hormone on its receptors from the liver membranes of salmonids e.g. *Salmo*

*salar* and *Oncorhynchus mykiss*.

The same recombinant microorganisms as described above but with an expression of the somatotropin representing at least 0.1% of the total protein, may be used as a "purified salmonid somatotropin preparation" when the product is to be delivered orally or by dipping and when the host microorganism itself may be added to the fish food or to the water.

An encapsulation or a chemical or a enzymatical modification of the ST may be used to facilitate its renaturation and purification, to change its affinity for its receptor, or to protect the hormone in the fish tissues against proteolytic attack and also in the case of an oral or dipping dosage against the denaturing and the proteolytic activities contained in the gastro-intestinal tract of the treated fish or in the water. Such methods are described for example by Mozhaev, Berezin and Martinek, CRC Critical reviews in Biochemistry, 23, 235:281, 1983. Such modifications will not prevent the *in vivo* binding of the hormone on its hepatic receptors. Whatever the modification it should not entail delayed release of the somatotropin in the blood circulation : e.g encapsulating materials should not be digestible in less time than the food absorbed by the fishes, when the administration is to be performed by the oral route and with the fish food.

EXAMPLE 1:

Recombinant trout GH production:

BL21 cells transformed with recombinant plasmid PARAE carrying trout growth hormone (rtGH) cDNA under lac promoter control were grown in bench fermentor with sufficient agitation and aeration to achieve a growth rate of about 70 min per cell division.

At a cell density of 2 g dry weight, IPTG was added to the culture at a concentration of 1 mM. After 3 to 6 hours, the cells become elongated and refractiles bodies containing rtGH could be seen under phase contrast microscope at 1000-fold magnification.

The cells were harvested by tangential filtration and the inclusion bodies isolated by centrifugation after disruption of the cells. The particules showed a protein band corresponding to a molecular weight of 22,000 daltons on 2-beta-mercaptoethanol SDS-PAGE.

After dissolving the particles in 8M guanidine chloride and dialysis in 0.02 N $NH_4HCO_3$, the recombinant protein was purified to homogeneity by ion-exchange chromatography and lyophilized. The biological activity of the hormone, as assessed by radioreceptor-assay using a trout liver membrane preparation, was equivalent to that of native salmonid GH (Fig 2).

Fish treatment:

Eight months-old atlantic salmons (25-35 g) reared in freshwater under natural conditions of temperature and photoperiod were randomly distributed in three groups of 50 fish before to be anaesthetised in 300 ppm phenoxyethanol.

Lyophilised rtGH was dissolved in saline buffer (PBS) and injected (10 $\mu$g rtGH in 250 $\mu$l) into the peritoneal cavity of the fish of rtGH treated group. Two additional groups were used as controls: the saline treated group received an intra peritoneal injection of 250$\mu$l PBS and the control group did not receive any treatment.

The fish were kept in freshwater for 12 more days before to be transferred into full salinity seawater (3.5 %; 10°C). The mortality recorded during the following 2 weeks was of 7.5 % for the rtGH treated group, and 100% for the saline injected and the untreated groups. The blood osmotic pressure measured in blood samples (10 samples per group) collected 24 hours after the transfer confirms that only rtGH treated salmons were able to control immediately their ionic balance in seawater (Table 1).

| | Mortality in % (14 days after transfer) | Blood osmotic pressure (24h after transfer) in milli-Osm/l |
|---|---|---|
| Control group | 100 % | 433± 10 |
| Saline injected group | 100 % | — |
| rtGH treated group | 7.5 % | 378± 6 |

**Table 1**: Effect of rtGH on the transfer of Atlantic salmon (8 months old; 25-35 g) into seawater.

EXAMPLE 2:

rtGH supraphysiological pulse:

The kinetics of disappearence of injected rtGH in salmonid blood were evaluated in conditions close to that of rtGH treatment reported in example 1.

Yearling rainbow trouts (30 g) were anaesthetised in 300 ppm phenoxyethanol and intraperitoneally injected with 10 $\mu$g rtGH in 250 $\mu$l PBS. The fish were kept in freshwater (7°C) under natural conditions (photoperiod and temperature) and blood samples (5 per point) were collected 2,4,6,8,12,16,24,48 and 72 hours after the injection.of rtGH. Control blood samples were collected from saline injected fish in parallel. Blood rtGH was assayed by a tGH specific radioimmunoassay developed and calibrated in house as described in Fryer et al.(Gen. Comp. Endocrinol. 39:123-130, 1979). The results of the kinetics are shown on Fig 3.

**Claims**

1. A process for the aquaculture of salmonids which comprises a farming step of these salmonids in a fresh water farm and another farming step in plain sea water for a time long enough to enable them to grow to the size finally sought, characterized by a single administration of somatotropin in a form readily releasable in the blood circulation of said farmed salmonids prior to their transfer from the fresh water farm into said plain sea water, in an amount sufficient to induce in their blood circulation a supraphysiological pulse of somatotropin effective to cause resumption of the growth of said salmonids to said size finally sought during said farming step in plain sea water.

2. The process of claim 1 wherein said transfer into seawater does not occur before the time at which said supraphysiological pulse reaches its maximum value.

3. The process of claim 1 wherein said transfer into seawater does not occur before the completion of said supraphysiological pulse and the return of the circulating levels of somatotropin back to substantially normal rates.

4. The process of any of claims 1 to 3 wherein said amount of somatotropin administered is sufficient to cause the peak of said physiological pulse in terms of somatotropin concentration in the blood circulation of said salmonids to temporarily exceed 50 ng per ml of blood.

5. The process of claim 4, wherein said amount of somatotropin is sufficient to cause said peak to temporarily exceed 300 ng, optionally even 750 ng per ml of blood.

7

6. The process of any of claims 1 to 5 wherein said physiological pulse is completed and the blood concentration of somatotropin is substantially back to normal rates after up to or less than three days after said administration.

7. The process of any of claims 1 to 6 wherein said single administration takes place at a time from 1 to 30 days, preferably from 3 to 20 days, e.g. from 10 to 14 days prior to the transfer of said salmonids in plain sea water.

8. The process of any of claim 1 to 7 wherein said somatotropin is a native or recombinant somatotropin homologous to the endogenous somatotropin of said salmonid.

9. The process of claim 8 , wherein said single administration is carried out by parenteral injection in an amount ranging from 30 ng to 30 $\mu$g per gram of fish, preferably from 0,10 to 0,25 $\mu$g per gram of fish.

10. The process of claim 8 or 9, wherein said administration is carried out orally, preferably in an an amount from 10 $\mu$g to 50 mg of somatotropin, e.g., per gram of fish food.

11. The process of claim 8, wherein said administration is carried out by dipping the fish in water containing from 25 mg to 10 g of somatotropin per liter of water for at least 30 seconds.

12. The process of claim 8, wherein said administration is carried out by adding the somatotropin in the water tank containing the fish, at a final concentration of somatotropin of 2,5 mg to 1 g per liter of water and maintaining the fishes in that medium for at least 30 minutes.

13. The process of any of claim 1 to 7 wherein said somatotropin is a native or recombinant heterologous somatotropin with respect to the endogenous somatotropin of the treated salmonid host, e.g. a mammalian somatotropin.

14. The process of claim 13 wherein said heterologous somatotropin is administered under the form of administration corresponding to that contemplated in any of claims 9 to 12, and in a amount corresponding to that of any of said claims 9 to 12 multiplied by a coefficient corresponding to the ratio of the affinity of a homologous somatotropin to the affinity of said heterologous somatotropin for somatotropin receptors as respectively measured in a radioreceptor-assay using natural receptors prepared from salmonid liver membranes.

15. The process of any of claims 1 to 14, wherein said salmonids species are selected among those which undergo smolting under natural conditions.

16. The process of any of claims 1 to 14, wherein said salmonids belong to the Salmo salar family.

17. The process of any of claims 1 to 14 wherein said salmonids belong to the Oncorhynchus family.

**Patentansprüche**

1. Verfahren zur Aquakultur von Salmoniden, welches die Haltung dieser Salmoniden in einer Süßwasserfarm und eine weitere Haltung in normalem Meerwasser für eine Zeit umfaßt, die lang genug ist, ihnen das Wachstum bis zur beabsichtigten Größe zu ermöglichen,
**gekennzeichnet** durch eine einzelne Verabreichung von Somatotropin in einer im Blutstrom der gehaltenen Salmoniden leicht freisetzbaren Form vor ihrem Transfer aus der Süßwasserfarm in das normale Meerwasser, in einer Menge, die ausreichend ist, in ihrem Blutkreislauf einen supraphysiologischen Somatotropinpuls auszulösen, der ausreichend ist, das Weiterwachstum der Salmoniden auf die letztendlich beabsichtigte Größe während des Haltens in normalem Meerwasser zu bewirken.

2. Verfahren nach Anspruch 1, worin der Transfer in das Meerwasser nicht vor dem Zeitpunk erfolgt, bei dem der supraphysiologische Puls seinen Maximalwert erreicht.

3. Verfahren nach Anspruch 1, worin der Transfer in das Meerwasser nicht vor Beendigung des supraphysiologischen Pulses und der Rückkehr des normalen Blutspiegels von Somatotropin zurück auf die im wesentlichen normalen Werte erfolgt.

4.  Verfahren nach einem der Ansprüche 1 bis 3, worin die Menge des zu verabreichenden Somatropins ausreichend ist, eine Spitze des physiologischen Pulses hinsichtlich der Somatropinkonzentration im Blutkreislauf der Salmoniden zu erzeugen, die temporär 50 ng pro ml Blut übersteigt.

5.  Verfahren nach Anspruch 4, worin die Menge des Somatotropins ausreichend ist, eine Spitze zu erzeugen, die temporär 300 ng, wahlweise sogar 750 ng pro ml Blut übersteigt.

6.  Verfahren nach einem der Ansprüche 1 bis 5, worin der physiologische Puls nach bis zu oder weniger als 3 Tagen nach der Verabreichung zum Erliegen kommt und die Blutkonzentration von Somatotropin im wesentlichen zurück auf normale Werte fällt.

7.  Verfahren nach einem der Ansprüche 1 bis 6, worin die Einzelverabreichung zu einem Zeitpunkt von 1 bis 30 Tagen, vorzugsweise 3 bis 20 Tagen, z.B. von 10 bis 14 Tagen vor dem Transfer der Salmoniden in das normale Meerwasser erfolgt.

8.  Verfahren nach einem der Ansprüche 1 bis 7, worin das Somatotropin ein natives oder rekombinantes Somatotropin ist, das homolog zum endogenen Somtotropin der Salmoniden ist.

9.  Verfahren nach Anspruch 8, worin die Einzelverabreichung durch parenterale Injektion in einer Menge im Bereich von 30 ng bis 30 $\mu$g pro Gramm Fisch, vorzugsweise von 0,10 bis 0,25 $\mu$g pro Gramm Fisch, erfolgt.

10. Verfahren nach Anspruch 8 oder 9, worin die Verabreichung oral, vorzugsweise in einer Menge von z.B. 10 $\mu$g bis 50 mg Somatotropin, pro Gramm Fischfutter, erfolgt.

11. Verfahren nach Anspruch 8, worin die Verabreichung durch Eintauchen des Fisches in Wasser, welches 25 mg bis 10 g Somatotropin pro Liter Wasser enthält, für mindestens 30 Sekunden durchgeführt wird.

12. Verfahren nach Anspruch 8, worin die Verabreichung durch Zugabe des Somatotropins in das Aquarium mit den Fischen in einer Endkonzentration des Somatotropins von 2,5 mg bis 1 g pro Liter Wasser und Halten der Fische in diesem Medium für mindestens 30 Minuten durchgeführt wird.

13. Verfahren nach einem der Ansprüche 1 bis 7, worin das Somatotropin natives oder rekombinantes heterologes Somatotropin in Bezug auf das endogene Somatotropin des behandelten Salmoniden, z.B. ein Säugersomatotropin, ist.

14. Verfahren nach Anspruch 13, worin das heterologe Somatotropin in der Verabreichungsform gemäß einem der Ansprüche 9 bis 12 und in einer Menge gemäß einem der Ansprüche 9 bis 12, multipliziert mit einem Koeffizienten entsprechend dem Verhältnis der Affinität des homologen Somatotropins zur Affinität des heterologen Somatotropins für die Somatotropin-Rezeptoren, wie entsprechend in einem Radiorezeptortest unter Verwendung natürlicher Rezeptoren, hergestellt aus Salmoniden-Lebermembranen gemessen, verabreicht wird.

15. Verfahren nach einem der Ansprüche 1 bis 14, worin die Salmonidenspezies ausgewählt sind aus solchen, die unter natürlichen Bedingungen als Junglachse in das Meer wandern.

16. Verfahren nach einem der Ansprüche 1 bis 14, worin die Salmoniden zur Salmo-Salar-Familie gehören.

17. Verfahren nach einem der Ansprüche 1 bis 14, worin die Salmoniden zur Oncorhynchus-Familie gehören.

**Revendications**

1.  Procédé de salmoniculture comprenant une étape d'élevage des salmonidés en eau fraîche et une autre étape d'élevage en pleine mer pendant une période suffisamment longue pour leur permettre de grandir jusqu'à la taille finale recherchée, caractérisé par une unique administration de somatotropine, sous une forme facilement assimilable par la circulation sanguine des dits salmonidés d'élevage, avant leur transfert depuis le bassin d'eau fraîche vers la pleine mer, en une quantité suffisante pour induire dans leur circulation sanguine une impulsion supraphysiologique de somatotropine, efficace pour provoquer, au cours de ladite étape d'élevage en pleine mer, la reprise de la croissance des dits salmonidés jusqu'à la taille finale recherchée.

2. Procédé selon la revendication 1, dans lequel ledit transfert vers l'eau de mer n'est pas effectué avant que ladite impulsion supraphysiologique n'atteigne sa valeur maximale.

3. Procédé selon la revendication 1, dans lequel ledit transfert vers l'eau de mer n'est pas effectué avant la fin de ladite impulsion supraphysiologique et le retour des taux de somatotropine dans la circulation à des valeurs sensiblement normales.

4. Procédé selon l'une quelconque des revendications 1 à 3, dans lequel ladite quantité de somatotropine administrée est suffisante pour amener le pic de ladite impulsion supraphysiologique, en termes de concentration en somatotropine dans la circulation sanguine des dits salmonidés, à dépasser temporairement une valeur de 50 ng par ml de sang.

5. Procédé selon la revendication 4, dans lequel ladite quantité de somatotropine est suffisante pour amener ledit pic à dépasser temporairement une valeur de 300 ng, voir même de 750 ng, par ml de sang.

6. Procédé selon l'une quelconque des revendications 1 à 5, dans lequel ladite impulsion supraphysiologique est terminée et la concentration en somatotropine dans le sang est globalement revenue à son taux normal au plus tard trois jours après ladite administration.

7. Procédé selon l'une quelconque des revendications 1 à 6, dans lequel ladite administration unique est effectuée entre 1 et 30 jours, de préférence entre 3 et 30 jours, par exemple entre 10 et 14 jours, avant le transfert des dits salmonidés vers la pleine mer.

8. Procédé selon l'une quelconque des revendications 1 à 7, dans lequel ladite somatotropine est une somatotropine naturelle ou de recombinaison homologue à la somatotropine endogène du dit salmonidé.

9. Procédé selon la revendication 8, dans lequel ladite administration unique est réalisée par injection parentérale d'une quantité allant de 30 ng à 30 $\mu$g par gramme de poisson, de préférence de 0,10 à 0,25 $\mu$g par gramme de poisson.

10. Procédé selon la revendication 8 ou la revendication 9, dans lequel ladite administration est réalisée par voie orale, de préférence en une quantité allant de 10 $\mu$g à 50 mg, par exemple, par gramme de chair de poisson.

11. Procédé selon la revendication 8, dans lequel ladite administration est réalisée en plongeant le poisson pendant au moins 30 secondes dans une eau contenant entre 25 mg et 10 g de somatotropine par litre d'eau.

12. Procédé selon la revendication 8, dans lequel ladite administration est réalisée en ajoutant de la somatotropine dans le bassin d'eau contenant les poissons, avec une concentration finale en somatotropine de 2,5 mg à 1 g par litre d'eau, et en maintenant les poissons dans ce milieu pendant au moins 30 minutes.

13. Procédé selon l'une quelconque des revendications 1 à 7, dans lequel ladite somatotropine est une somatotropine naturelle ou de recombinaison hétérologue par rapport à la somatotropine endogène du salmonidé traité, par exemple la somatotropine d'un mammifère.

14. Procédé selon la revendication 13, dans lequel ladite somatotropine hétérologue est administrée sous la forme d'une administration équivalente à celle décrite dans l'une quelconque des revendications 9 à 12, multipliée par un coefficient correspondant au rapport de l'affinité d'une somatotropine homologue à l'affinité de ladite somatotropine hétérologue pour les récepteurs de somatotropine, toutes deux mesurées respectivement par une analyse de radiorécepteur utilisant des récepteurs naturels préparés à partir de la membrane du foie des salmonidés.

15. Procédé selon l'une quelconque des revendications 1 à 14, dans lequel l'espèce des dits salmonidés est choisie parmi celles qui connaissent une smoltification dans les conditions naturelles.

16. Procédé selon l'une quelconque des revendications 1 à 14, dans lequel lesdits salmonidés font partie de la famille des salmo salar.

17. Procédé selon l'une quelconque des revendications 1 à 14, dans lequel lesdits salmonidés font partie de la famille des oncorhynchus.

Figure 1
Somatotropin concentration in serum of
rainbow trout (20-100g)

Figure 2

**Recombinant trout growth hormone (rtGH)**
RIA after i.p. injection of 10 µg rtGH

+ **Injected**    • Controls

**5 fish per point**

fig 3

Growth Hormone RRA
Trout liver GH receptors